Europäisches Patentamt

European Patent Office

Office européen des brevets

(11) Numéro de publication: **0 304 631 B1**

(12) **FASCICULE DE BREVET EUROPEEN**

(45) Date de publication de fascicule du brevet: **25.03.92**   (51) Int. Cl.⁵: **A61F  13/15**

(21) Numéro de dépôt: **88111836.8**

(22) Date de dépôt: **22.07.88**

(54) **Couche-culotte à jeter à élastiques d'entre-jambe et enduction latérales d'étanchéité.**

(30) Priorité: **30.07.87 FR 8710834**

(43) Date de publication de la demande:
**01.03.89 Bulletin  89/09**

(45) Mention de la délivrance du brevet:
**25.03.92 Bulletin  92/13**

(84) Etats contractants désignés:
**AT BE CH DE ES FR GB GR IT LI LU NL SE**

(56) Documents cités:
**EP-A- 0 113 976        EP-A- 0 196 654**
**EP-A- 0 270 979        DE-A- 3 319 043**
**US-A- 3 799 167        US-A- 4 610 685**

(73) Titulaire: **PEAUDOUCE**
**59, Rue de la Vignette**
**F-59126 Linselles(FR)**

(72) Inventeur: **Leroy, André**
**1, Allée des Glycines**
**F-59420 Mouvaux(FR)**
Inventeur: **Villez, Yves**
**13, Rue Albert Camus**
**F-59126 Inselles(FR)**

(74) Mandataire: **Casalonga, Axel et al**
**BUREAU D.A. CASALONGA - JOSSE Morassistrasse 8**
**W-8000 München 5(DE)**

## Description

La présente invention se rapporte à des couches-culottes à jeter pour enfants ou adultes incontinents. Les couches-culottes du type de l'invention comprennent une enveloppe extérieure imperméable aux liquides qui peut être réalisée avantageusement à partir d'une feuille souple mince imperméable, par exemple en polyéthylène. Un matelas absorbant est fixé à l'intérieur de l'enveloppe imperméable, en retrait par rapport aux bords extrêmes de l'enveloppe imperméable de façon à laisser subsister de chaque côté du matelas absorbant une bordure latérale. Le matelas absorbant peut comporter avantageusement de la pulpe de cellulose défibrée couramment appelée "fluff" dans la technique ainsi qu'éventuellement des particules ou grains de matériau superabsorbant c'est-à-dire de matériau polymère capable d'absorber plusieurs fois son volume de liquide. Une feuille perméable interne recouvre en général la face interne de l'enveloppe imperméable et le matelas absorbant. Cette feuille perméable est habituellement un voile en non-tissé capable de laisser passer les liquides afin que ceux-ci puissent pénétrer dans le matelas absorbant. Le matelas absorbant est fixé, avantageusement par collage, sur la majeure partie de sa surface par exemple au moyen de lignes d'encollage longitudinales. La feuille perméable est également fixée par collage, par exemple au moyen de lignes longitudinales de colle sur la surface supérieure du matelas absorbant et sur les zones de bordure latérale et sur les zones avant et arrière de l'enveloppe imperméable qui déborde du matelas absorbant. Au moins un élément élastique latéral est fixé par collage à l'état tendu dans une zone transversale médiane de chaque bordure latérale de l'enveloppe imperméable. Deux rubans de feuille souple sont fixés à l'enveloppe imperméable de façon que chaque ruban enserre un élément élastique latéral dans une gaine isolante s'étendant sur toute la longueur de l'enveloppe imperméable.

Lors de la fabrication de telles couches-culottes individuelles, les éléments élastiques sont appliqués à l'état tendu et en continu sur la feuille souple destinée à constituer l'enveloppe imperméable des couches-culottes. Ces éléments élastiques sont encollés de manière discontinue de sorte que seules leurs sections encollées adhèrent à la feuille imperméable. En fin de fabrication, on procède à une découpe transversale de la bande fabriquée, cette découpe étant faite entre les matelas absorbants successifs de façon à définir les couches-culottes individuelles. Lors de cette découpe transversale, les sections non encollées des éléments élastiques se rétractent librement à l'intérieur des gaines isolantes formées par les rubans de feuille souple. L'existence de ces gaines isolantes permet d'éviter l'échappement vers l'extérieur, à l'endroit des bords transversaux de la couche-culotte, des liquides absorbés par le matelas de la couche-culotte. De plus grâce à l'existence de ces gaines isolantes, la matière constitutive du matelas absorbant et en particulier d'éventuelles particules de superabsorbant disposées à l'intérieur de la couche-culotte sur la face interne de l'enveloppe imperméable, ne peuvent pas pénétrer dans les gaines ainsi définies et s'échapper vers l'extérieur du fait que ces gaines ou tunnels ne sont ouverts qu'aux deux bords extrêmes transversaux de la couche-culotte.

Dans les couches-culottes habituelles connues jusqu'à présent, on a cherché à résoudre le problème de la migration des liquides contenus dans le matelas absorbant vers l'extérieur. C'est ainsi que l'on a déjà prévu de disposer au voisinage des bordures transversales avant et arrière de la couche-culotte des éléments formant barrière capables d'empêcher la remontée des liquides depuis le matelas absorbant vers la zone de ceinture de la couche-culotte en freinant la migration des liquides dans les zones latérales de la couche-culotte, par l'intermédiaire de la feuille perméable qui recouvre la totalité de la surface interne de la couche-culotte. C'est ainsi que les brevets US 3 520 303 (Endres), US 3 606 422 (Sabee) et US 3 386 442 (Sabee) décrivent des couches-culottes, dénuées d'élastiques d'entre-jambe, mais comportant des rubans de matière plastique recouvrant les bordures latérales du matelas absorbant. Le brevet US 3 799 167 (Miller) prévoit de traiter les zones périphériques d'un matelas absorbant avec une émulsion de polymère qui est soumise à traitement thermique de durcissement après application. Aucun élastique d'entre-jambe n'est envisagé dans ce document.

Toutes ces solutions antérieures, si elles paraissent en théorie devoir résoudre le problème de l'étanchéité latérale ne peuvent cependant pas être appliquées dans la fabrication en grande série de couches-culottes à jeter. De plus elles sont totalement inappropriées au cas de couches-culottes comportant des élastiques d'entre-jambe. La demande de brevet européen EP 59014 (Procter et Gamble) qui décrit une couche-culotte à élastiques d'entre-jambe a tenté de résoudre la difficulté en pratiquant des lignes longitudinales de compression de matière parallèlement aux élastiques afin de freiner la migration des liquides. Une telle réalisation, outre qu'elle ne fournit qu'une étanchéité relative ne permet pas d'éviter une remontée des liquides directement par le matelas absorbant.

D'autres solutions ont été préconisées pour réaliser une sorte de fenêtre à bords imperméables au moyen de l'adjonction d'une feuille mince de matière plastique à l'intérieur même de la couche-culotte. Les brevets FR 2 181 792 (Paper Converting), FR 2 299 824 (CGT) et FR 2 522 521 (Béghin-Say) décrivent des couches-culottes de ce type qui n'ont pas donné entière satisfaction dans la pratique. De plus de telles réalisations sont de fabrication complexe étant donné qu'il est nécessaire de rajouter des éléments de feuille après découpe appropriée et d'affecter un collage convenable supplémentaire à l'intérieur même de la couche-culotte.

La présente invention a pour objet une couche-culotte du type mentionné dans le préambule de la description, dans laquelle la migration des liquides depuis le matelas absorbant vers l'extérieur dans les zones latérales soit supprimée ou très largement diminuée et ce sans influer sur l'action des éléments élastiques qui peuvent ainsi continuer à assurer leur rôle d'adaptation de la couche-culotte à la morphologie de l'utilisateur et d'amélioration de l'étanchéité au passage des jambes. Il convient en effet de prévoir, au moment de la fabrication, des moyens permettant le passage des éléments élastiques latéraux et n'empêchant pas le retrait de leurs portions d'extrémité non encollées lors de la découpe finale des couches-culottes individuelles.

La couche-culotte à jeter pour enfants ou adultes incontinents selon la présente invention est du type comprenant une enveloppe extérieure imperméable aux liquides, un matelas absorbant fixé à l'intérieur de l'enveloppe imperméable, en retrait par rapport aux bords extrêmes de l'enveloppe imperméable de façon à laisser subsister de chaque côté du matelas une bordure latérale, une feuille perméable interne recouvrant la face interne de l'enveloppe imperméable et le matelas absorbant, au moins un élément élastique latéral fixé par collage à l'état tendu dans une zone transversale médiane de chaque bordure latérale de l'enveloppe imperméable et deux rubans de feuille souple fixés à l'enveloppe imperméable de façon que chaque ruban enserre un élément élastique latéral dans une gaine isolante s'étendant sur toute la longueur de l'enveloppe imperméable. Selon l'invention, les zones latérales de la feuille perméable interne sont recouvertes, de préférence sur leur face dirigée vers l'extérieur de la couche-culotte, d'une enduction d'un matériau d'étanchéité liquide à haute température et formant après refroidissement dans l'épaisseur de ladite feuille, une barrière à la propagation des liquides, ladite enduction étant faite jusque dans les portions de la feuille perméable qui recouvrent les bordures latérales du matelas absorbant et sur toute la longueur de la couche-culotte. On entend par "face dirigée vers l'extérieur de la couche-culotte" ou face externe, la face de la feuille perméable interne qui vient en contact avec le matelas absorbant. L'enduction s'étend en partie dans l'épaisseur de la feuille perméable sans toutefois traverser complètement ladite feuille. La face interne de la feuille perméable garde donc un toucher et un état de surface doux pour le contact avec la peau de l'utilisateur.

L'enduction s'étend de préférence jusqu'aux bords latéraux extrêmes de la couche-culotte.

Dans un mode de réalisation avantageux de l'invention, les rubans des gaines isolantes sont fixés sur la face interne de l'enveloppe imperméable et enserrent les éléments élastiques également fixés par collage sur la face interne de l'enveloppe imperméable. Dans ce cas, les rubans sont de préférence réalisés en feuille mince imperméable aux liquides, par exemple en polyéthylène.

Grâce à l'existence de ces rubans en feuille mince imperméable, le matériau liquide à haute température qui vient pénétrer l'épaisseur de la feuille perméable interne ne risque pas d'entrer en contact avec les éléments élastiques et en particulier avec leurs extrémités non encollées, puisque les éléments élastiques sont séparés de la feuille perméable interne par la gaine isolante formée par le ruban en feuille souple imperméable qui les recouvre. Grâce à cette disposition, il devient donc possible d'enduire sans difficulté la face externe de la feuille perméable dans ses zones latérales de façon à obtenir un effet de barrière à la propagation des liquides dans la zone du passage des jambes, sans interférer avec le processus de fabrication de la couche-culotte et en particulier sans gêner en quoi que ce soit le retrait des extrémités libres non encollées des éléments élastiques latéraux lors de la découpe qui forme les couches-culottes individuelles.

Dans un autre mode de réalisation, les rubans des gaines isolantes sont fixés sur la face externe de l'enveloppe imperméable et viennent enserrer les éléments élastiques également fixés par collage sur la face externe de l'enveloppe imperméable. Dans ce mode de réalisation, il n'est pas indispensable que le ruban soit réalisé en une matière imperméable et l'on peut envisager de réaliser ce ruban en toute autre matière telle que du non-tissé. Comme dans le mode de réalisation précédent, les éléments élastiques se trouvent totalement isolés du matériau d'enduction qui vient pénétrer à l'intérieur de l'épaisseur de la feuille perméable interne, l'isolation se faisant cette fois par l'enveloppe imperméable extérieure elle-même. On obtient donc les mêmes avantages que dans le mode de réalisation précédent.

Il apparaît que l'invention dans la combinaison de l'enduction du matériau d'étanchéité liquide à haute température formant après refroidissement une barrière à la propagation des liquides à l'intérieur même de l'épaisseur de la feuille perméable interne, avec les rubans fixés à l'enveloppe imperméable et réalisant une gaine isolante s'étendant sur toute la longueur de l'enveloppe imperméable enserrant les éléments élastiques latéraux.

La structure des éléments élastiques latéraux n'est pas critique en elle-même pour la présente invention. C'est ainsi que l'on peut utiliser des éléments élastiques constitués chacun par une bandelette en matière élastique, par exemple en caoutchouc ou toute autre matière. On peut également utiliser des éléments élastiques constitués par une pluralité de brins individuels placés parallèlement les uns aux autres éventuellement par groupes.

En général et pour des raisons de simplification du procédé de fabrication, on utilisera des éléments élastiques collés à l'état rectiligne. On pourrait cependant parfaitement envisager d'utiliser des éléments élastiques curvilignes dans la mesure où la gaine isolante présenterait des dimensions capables de recevoir les éléments élastiques non rectilignes.

Dans toute la description de l'invention on qualifie d'"externes" les éléments constitutifs dirigés vers l'extérieur lorsque la couche-culotte est portée par l'utilisateur et d'"internes" les éléments constitutifs se trouvant entre l'utilisateur et la feuille imperméable.

La présente invention sera mieux comprise à l'étude de quelques modes de réalisation décrits à titre d'exemples nullement limitatifs et illustrés par les dessins annexés sur lesquels :

la figure 1 est une vue de dessus avec arrachement d'une couche-culotte conforme à l'invention montrée à l'état tendu et à plat la face interne vers le haut;

la figure 2 est une vue partielle en coupe selon II-II de la figure 1 les épaisseurs des différents matériaux ayant été fortement exagérées;

la figure 3 est une vue analogue à la figure 2 d'une variante de réalisation utilisant des élastiques comportant une pluralité de brins individuels; et

la figure 4 est une vue analogue aux figures 2 et 3 montrant une autre variante de l'invention dans laquelle les éléments élastiques sont fixés à l'extérieur de l'enveloppe imperméable.

Telle qu'elle est illustrée sur les figures 1 et 2, la couche-culotte selon l'invention comprend une enveloppe extérieure 1 imperméable aux liquides, réalisée à partir d'une feuille mince, souple par exemple en polyéthylène. L'enveloppe 1 comprend une portion transversale arrière 2, une portion transversale avant 3 qui est avantageusement de même largeur que la partie transversale 2 bien qu'une largeur différente puisse être envisagée et une portion centrale 4 de largeur inférieure, obtenue par découpe latérale d'échancrure 5 à l'endroit du passage des jambes lors de l'utilisation de la couche-culotte. La portion centrale 4 ou zone d'entre-jambes est délimitée dans l'exemple illustré par un bord sensiblement rectiligne 6 parallèle à l'axe longitudinal de la couche-culotte.

Sur toute la surface interne de l'enveloppe imperméable 1 ont été appliquées en continu de fines lignes de collage 7 parallèles entre-elles. Cette opération effectuée en continu sur la feuille mince en polyéthylène destinée à constituer l'enveloppe est effectuée bien entendu avant la découpe des échancrures latérales 5. Deux éléments élastiques 8 disposés de façon rectiligne parallèlement à l'axe longitudinal de la couche-culotte ont été encollés sur leur portion médiane 9 entre deux lignes de collage 7, cette opération se faisant à l'état tendu des éléments élastiques 8. Les portions d'extrémité avant et arrière 10 des éléments élastiques 8 sont au contraire dénuées de tout adhésif de sorte que, lors de la découpe transversale finale de la bande continue complète destinée à former les couches-culottes individuelles, ces portions d'extrémité se rétractent en laissant apparaître des bouts libres non tendus visibles sur la figure 1. Dans l'exemple illustré sur les figures 1 et 2, chacun des éléments élastiques 8 est constitué par une bandelette de section sensiblement rectangulaire réalisée par exemple en caoutchouc. On comprendra bien entendu que d'autres matériaux pourraient être employés et que la section de l'élément élastique pourrait être modifiée sans difficulté l'essentiel étant la fixation de la portion médiane de l'élément élastique à l'état tendu sur l'enveloppe imperméable 1. De la même manière, dans l'exemple illustré, on a prévu la pose des éléments élastiques avec une tension constante, les portions d'extrémité 10 n'étant pas encollées. On pourrait parfaitement envisager au contraire d'encoller en continu les éléments élastiques 8 en faisant varier la tension au moment de la pose de façon que les extrémités avant et arrière des éléments élastiques 8 soient posées sans tension sur l'enveloppe imperméable 1.

De la même manière si l'on a illustré ici des éléments élastiques posés selon une ligne continue et rectiligne, on pourrait envisager dans d'autres modes de réalisation d'utiliser des éléments élastiques posés par portion, discontinues ou selon une ligne courbe, par exemple suivant le profil sensiblement courbe des échancrures latérales 5.

Un matelas absorbant 11 est posé à l'intérieur de l'enveloppe imperméable 1 en étant fixé sur cette dernière par les multiples lignes de collage longitudinales 7. Comme on peut le voir sur la figure 1, le matelas absorbant est fixé en retrait par rapport aux bords extrêmes de l'enveloppe imperméable 1 de façon à laisser subsister de chaque côté du matelas une bordure latérale 12 de la portion médiane 4 de l'enveloppe imperméable 1, les éléments élastiques 8 se trouvant disposés sur cette bordure latérale 12. Dans l'exemple illustré, le matelas absorbant 11 présente une forme générale de sablier avec une portion transversale arrière 13, une portion transversale avant 14 et une portion médiane 15 de largeur inférieure. Les éléments élastiques 8 sont de préférence encollés dans leur portion médiane 9 jusqu'au voisinage du bord du matelas absorbant 11 dans sa partie curviligne qui rejoint la portion médiane 15 et les portions transversales 13 et 14.

Le matelas absorbant 11 est de préférence constitué de pulpe de cellulose défibrée présentant un caractère absorbant important. Dans un mode de réalisation préféré on peut également adjoindre à la matière absorbante ainsi définie une matière synthétique superabsorbante constituée par des polymères insolubles dans l'eau, formant un gel, tels que des hydrocolloïdes capables d'absorber de grandes quantités de liquides et de retenir les liquides ainsi absorbés. On utilisera par exemple des polymères d'acides carboxyliques non saturés tels que l'acide acrylique, ces polymères étant rendus insolubles dans l'eau par réticulation partielle des groupes carboxyliques par des agents réticulants de type classique. On pourra se reporter par exemple au brevet US 4 654 039 (Brandt) qui cite de nombreux exemples de tels polymères de l'art antérieur. Cette matière superabsorbante peut être distribuée de manière uniforme sous forme de particules ou de grains à l'intérieur du matelas absorbant ou encore être répartie par couche entre des couches séparées du matelas absorbant 11.

Deux rubans 16 en feuille souple mince par exemple en polyéthylène, sont en outre fixés par des lignes de collage 7 ou d'autres lignes de collage plus serrées, sur la face interne de l'enveloppe imperméable 1 après la pose des éléments élastiques 8. Les rubans 16 posés parallèlement à l'axe longitudinal de la couche-culotte, sur toute sa longueur, au-dessus de chacun des éléments élastiques 8 définissent une sorte de gaine isolante ou de tunnel qui enserre l'élément élastique 8 sur toute la longueur de l'enveloppe imperméable 1. Lors de la découpe finale transversale définissant les couches-culottes, les extrémités 10 des éléments élastiques 8 subissent le retrait qui a été mentionné précédemment, à l'intérieur de la gaine isolante ainsi définie les portions non encollées de l'élastique pouvant glisser librement à l'intérieur de cette gaine.

Comme on pourra le noter sur les figures, le matelas absorbant 11 est en partie posé sur les rubans 16 en particulier dans la zone des extrémités transversales 13 et 14. Les rubans 16 peuvent également être disposés sous le matelas absorbant dans sa zone médiane 15 bien que cette disposition ne soit pas critique. L'essentiel en effet réside dans l'isolation de la gaine constituée par le ruban 16, cette isolation étant réalisée par les lignes de collage longitudinales 7 qui assurent la fixation des rubans 16 sur la face interne de l'enveloppe imperméable 1. Grâce à cet isolement, les tunnels ou gaines ainsi formés pour le passage des éléments élastiques 8, en particulier dans la zone de leurs extrémités avant et arrière 10, ne peuvent plus entrainer l'échappement vers l'extérieur des liquides par draînage de l'urine absorbée par le coussin. De la même manière tout échappement vers l'extérieur de la matière constitutive du coussin absorbant est évité, notamment dans le cas où le matelas absorbant 11 comprend des grains ou particules de superabsorbant qui peuvent se détacher de la pulpe de cellulose défibrée.

Les rubans 16 peuvent être réalisés en toute matière appropriée, par exemple en non-tissé à base de fibres de cellulose incluant éventuellement des fibres de matière plastique telles que polypropylène etc. Dans l'exemple illustré sur les figures 1 et 2 où les rubans 16 sont fixés par collage sur la face interne de l'enveloppe imperméable 1, les rubans 16 sont de préférence réalisés en un matériau imperméable tel que par exemple en polyéthylène dont l'épaisseur pourra être approximativement égale à celle de l'enveloppe extérieure 1 de façon à assurer une souplesse comparable.

La surface interne de la couche-culotte est entièrement recouverte d'une feuille perméable interne 17 disposée sur la face interne de l'enveloppe extérieure 1 et fixée par les lignes de collage longitudinales 7 tout autour du matelas absorbant 11. La feuille perméable 17 recouvre donc à la fois des portions de bordure de la feuille imperméable 1, des portions des rubans 16 et la totalité du matelas absorbant 11. La feuille perméable 18 peut être réalisée avantageusement en un voile de non-tissé perméable à base de fibres de cellulose ou contenant en outre des fibres de matière synthétique telle que du polyester éventuellement en mélange avec du nylon ou du polypropylène.

La couche-culotte comporte encore dans la partie transversale arrière 2 deux dispositifs d'attaches adhésives 18 de type classique permettant de maintenir fermée la couche-culotte sur l'utilisateur.

Les zones latérales longitudinales de la feuille perméable 17 sont recouvertes sur leur face externe, dirigée vers la feuille imperméable 1, d'une enduction 19 d'un matériau d'étanchéité. Un tel matériau dit "hot melt" dans la technique habituelle est liquide à haute température de l'ordre de 100°C et forme après refroidissement une couche non collante sensiblement imperméable aux liquides. Cette couche ainsi enduite pénètre en partie dans l'épaisseur même de la feuille perméable 17 comme on l'a représenté très schématiquement et de façon exagérée sur la figure 2, et sans toutefois traverser complètement la feuille 17 qui garde son toucher doux pour le contact avec la peau de l'utilisateur. L'enduction latérale 19 constitue une barrière à la propagation des liquides. L'urine ayant pénétré dans le matelas absorbant 11 ne peut donc plus migrer par l'intermédiaire des portions latérales du voile perméable 17 en direction de l'extérieur. Sur la figure 1, on a représenté schématiquement l'enduction 19 à l'extérieur de la feuille 17 au moyen de pointillés. Dans la réalité bien entendu, l'enduction 19 ne se voit pas de l'intérieur. Il y a lieu de noter que l'on peut aussi envisager de placer l'enduction 19 vers l'intérieur à condition de choisir un matériau d'enduction non collant à froid.

Les enductions latérales 19 sont pratiquées sur toute la longueur de la couche-culotte de façon à constituer non seulement une barrière à la migration latérale du liquide mais également à réaliser dans une certaine mesure un scellement d'extrémité des gaines isolantes assurant le passage des éléments élastiques 8. A cet effet on comprend que l'enduction 19 se fait au-dessus de la zone qui comporte les rubans 16, ceux-ci empêchant cependant par leur présence, toute pénétration du matériau d'étanchéité enduit jusqu'à l'intérieur des gaines isolantes. Dans ces conditions, l'enduction d'étanchéité devient possible malgré l'existence des portions d'extrémité 10 des éléments élastiques 8. Jusqu'à présent en effet, il n'avait pas été possible dans la pratique, de réaliser de telles barrières d'étanchéité latérales par enduction de "hot-melt" dans des couches-culottes comportant des éléments élastiques au passage des jambes en raison des difficultés rencontrées pour maintenir libres les extrémités des élastiques. Grâce à la présente invention, les éléments élastiques 8 sont totalement isolés par les rubans 16 qui s'étendent sur toute la longueur de la couche-culotte et il devient possible d'enduire les portions latérales de la feuille perméable 17 sur toute la longueur de la couche-culotte en assurant ainsi une excellente barrière latérale à la propagation des liquides.

L'enduction latérale 19 est de préférence réalisée jusque dans les portions de la feuille perméable 17 qui recouvrent les bords latéraux du matelas absorbant 11 de façon à créer en quelque sorte au-dessus dudit matelas absorbant, une bande longitudinale perméable délimitée par deux bordures imperméables constituées par les enductions 19. Dans le mode de réalisation illustré, les enductions 19 ont été faites jusqu'au bord extrême latéral de la couche-culotte. On pourrait cependant envisager de limiter l'enduction à une bande longitudinale recouvrant sensiblement la zone des rubans 16 sur toute la longueur de la couche-culotte.

A titre d'exemples de matériau d'étanchéité liquide à haute température que l'on peut utiliser dans la présente invention on peut citer : le hot melt de référence 5092-3-1 commercialisé par la Société "National adhesives and resins" appliqué à environ 135°C; le hot melt de référence 8698-336 commercialisé par la Société "Findley" appliqué à environ 135°C; le hot melt de référence 2-2271 commercialisé par la Société "Malcolm Nicol" appliqué à environ 145°C.

L'enduction du matériau d'étanchéité liquide à haute température ("hot-melt") peut se faire par tout dispositif approprié. On utilisera de préférence un dispositif de couchage à l'aide d'une filière capable de déposer le matériau d'étanchéité liquide sur la bande de matériau en défilement continu destiné à réaliser les couches-culottes après découpe transversale. A titre d'exemple on pourra utiliser les dispositifs vendus par la Société Acumeter Laboratories. On peut également utiliser un rouleau applicateur de hot melt ou encore des buses de pulvérisation.

La figure 3 illustre un mode de réalisation analogue au mode de réalisation précédent les éléments identiques portant les mêmes références. Dans ce mode de réalisation cependant, les éléments élastiques 8 ont été remplacés par un groupe de quatre brins élastiques individuels 20 disposés parallèlement les uns aux autres et encollés comme précédemment dans leur portion médiane. Les brins élastiques 20 présentent chacun une section sensiblement circulaire. Le ruban 16 qui recouvre l'ensemble des brins élastiques 20 assure comme précédemment la formation d'une gaine isolante pour les éléments élastiques. Les brins élastiques individuels peuvent être réalisés en latex ou en tout autre matériau élastique approprié.

Dans le mode de réalisation de la figure 4 où les éléments identiques portent les mêmes références, on a disposé quatre brins élastiques individuels 20 non plus sur la face interne de l'enveloppe imperméable 1 mais sur sa face externe, sensiblement dans la même position générale que sur la figure 3. Les rubans 16 sont alors fixés par collage également sur la face externe de l'enveloppe imperméable 1 et sur toute sa longueur, en constituant comme précédemment, avec l'enveloppe imperméable 1, une gaine isolante qui reçoit les brins élastiques 20. On notera que l'on obtient le même résultat que dans les modes de réalisation précédents, les liquides ne pouvant pas s'échapper par l'un quelconque des tunnels formés par le passage des éléments élastiques puisque ces derniers sont encollés sur la face externe de l'enveloppe imperméable 1.

Comme précédemment, l'enduction 19 ne peut interférer avec l'action de retrait des éléments élastiques 20 lors de la découpe transversale des couches-culotte grâce à l'interposition de l'enveloppe imperméable 1.

Dans ce mode de réalisation, il n'est pas nécessaire d'utiliser pour les rubans 16, une matière imperméable et seules les qualités de surface du matériau utilisé entreront en ligne de compte.

**Revendications**

1. Couche-culotte à jeter pour enfants ou adultes incontinents du type comprenant une enveloppe extérieure (1) imperméable aux liquides, un matelas absorbant (11) fixé à l'intérieur de l'enveloppe imperméable, en retrait par rapport aux bords externes de l'enveloppe imperméable, de façon à laisser subsister de chaque côté du matelas une bordure latérale, une feuille perméable interne (17) recouvrant la face interne de l'enveloppe imperméable et le matelas absorbant, au moins un élément élastique latéral (8, 20) fixé par collage à l'état tendu dans une zone transversale médiane de chaque bordure latérale de l'enveloppe imperméable et deux rubans (16) de feuille souple fixés à l'enveloppe imperméable de façon que chaque ruban enserre un élément élastique latéral dans une gaine isolante s'étendant sur toute la longueur de l'enveloppe imperméable, caractérisé par le fait que les rubans (16) s'étendent longitudinalement sur toute la longueur de la couche-culotte, les gaines isolantes étant ouvertes à leurs extrémités; que les extrémités des éléments élastiques (8, 20) ne sont pas encollées et subissent de ce fait un retrait à l'intérieur desdites gaines isolantes; et que les zones latérales de la feuille perméable interne sont recouvertes d'une enduction (19) d'un matériau d'étanchéité liquide à haute température et formant, après refroidissement, dans l'épaisseur de ladite feuille perméable, une barrière à la propagation des liquides, ladite enduction étant faite jusque dans les portions de la feuille perméable qui recouvrent les bordures latérales du matelas absorbant en recouvrant également les zones correspondant aux gaines isolantes et sur toute la longueur de la couche-culotte.

2. Couche-culotte à jeter selon la revendication 1, caractérisée par le fait que l'enduction (19) s'étend jusqu'aux bords latéraux extrêmes de la couche-culotte.

3. Couche-culotte à jeter selon les revendications 1 ou 2, caractérisée par le fait que les rubans (16) des gaines isolantes sont fixés sur la face interne de l'enveloppe imperméable (1) et enserrent les éléments élastiques (8, 20) également fixés par collage sur la face interne de l'enveloppe imperméable (1).

4. Couche-culotte à jeter selon la revendication 3, caractérisée par le fait que les rubans (16) sont réalisés en feuille imperméable aux liquides.

5. Couche-culotte à jeter selon l'une des revendications 1 ou 2, caractérisée par le fait que les rubans (16) des gaines isolantes sont fixés sur la face externe de l'enveloppe imperméable (1) et enserrent les éléments élastiques également fixés par collage sur la face externe de l'enveloppe imperméable (1).

6. Couche-culotte à jeter selon l'une quelconque des revendications précédentes, caractérisée par le fait que les éléments élastiques sont constitués chacun par une bandelette en matériau élastique.

7. Couche-culotte à jeter selon l'une quelconque des revendications 1 à 5, caractérisée par le fait que les éléments élastiques sont constitués chacun par une pluralité de brins individuels placés parallèlement les uns aux autres.

8. Couche-culotte à jeter selon l'une quelconque des revendications précédentes, caractérisée par le fait que les éléments élastiques sont fixés à l'état tendu par encollage dans leur portion médiane, leurs extrémités avant et arrière non fixées étant sans tension et rétractées à l'intérieur dans gaines isolantes précitées.

9. Couche-culotte à jeter selon l'une quelconque des revendications précédentes, caractérisée par le fait que les éléments élastiques sont rectilignes.

10. Couche-culotte à jeter selon l'une quelconque des revendications précédentes, caractérisée par le fait que le matelas absorbant comprend de la pulpe de cellulose défibrée et des grains de matière synthétique superabsorbante.

**Claims**

1. A disposable napkin for children or incontinent adults, of the type comprising an outer cover (1) which is impermeable to liquids, an absorbent padding (11) which is secured to the inside of the impermeable cover and which is set back relative to the outer edges of the impermeable cover so as to leave a lateral border on each side of the padding, an inner permeable sheet (17) covering the inner surface of the impermeable cover and the absorbent padding, at least one lateral elastic element (8, 20) attached by adhesive while under